# EUROPEAN PATENT APPLICATION

(11) **EP 4 523 684 A1**
(43) Date of publication of application: **19.03.2025**
(21) Application number: 23382935.7
(22) Date of filing: 14.09.2023
(51) Int. Cl.: A61K 31/202, A61K 31/557, A61P 1/00, A61P 19/02, A61P 29/00

(54) **COMPOSITIONS COMPRISING LIPOXIN-A4 AND MARESIN-1, AND USES THEREOF**

(71) Applicant: Fundacion Instituto De Investigacion Sanitaria Fundacion Jimenez Diaz, 28040 Madrid (ES)
(72) Inventor: LARGO CARAZO, Raquel, 28040 Madrid (ES); HERRERO-BEAUMONT CUENCA, Gabriel, 28040 Madrid (ES); BERMEJO ÁLVAREZ, Ismael, 28040 Madrid (ES); FUENTE PÉREZ, Rocío, 28040 Madrid (ES)
(74) Representative: Hoffmann Eitle

(57) **Abstract**

The present invention relates to a composition comprising Lipoxin A4 and Maresin 1, and its use in the treatment of diseases or conditions that involve inflammation, preferably acute inflammation.

## Description

### TECHNICAL FIELD OF THE INVENTION

The present invention relates to the field of medicine. In particular, the present invention relates to the field of compositions comprising Lipoxin-A4 and Maresin-1 and uses thereof.

### BACKGROUND OF THE INVENTION

Acute inflammation is a crucial and natural response of the body's immune system to harmful stimuli, such as infections, injuries, or tissue damage. This defensive mechanism aims to isolate and eliminate the irritant, as well as initiate the healing process. The hallmark signs of acute inflammation include redness, swelling, heat, pain, and impaired function in the affected area. The process involves the release of various inflammatory mediators, recruitment of immune cells, and increased blood flow to the site of injury or infection. Although essential for tissue repair, chronic or unresolved acute inflammation can lead to detrimental effects on the body and may contribute to the development of various diseases.

In fact, chronic diseases are characterized by an exacerbated inflammation where the body loses the ability of resolving it, resulting in a severe loss of homeostasis.

Arthritis refers those disorders that affects joints, and the symptoms generally include inflammation in one or more joints, pain, and stiffness. Microcrystal arthritis or arthropathy is a class of joint disorder that is characterized by accumulation of tiny crystals in one or more joints, causing inflammation. Even in the context of a chronic disease, acute accumulation of microcrystals of different composition in the joint causes an acute inflammatory process which is usually self-limited and able to be resolved within days, although causing severe pain and disability. While the standard treatment for those diseases is NSAIDs or glucocorticoids, these drugs can cause a delay in the inflammatory flare resolution and the tissue repair, which may enhance the risk of flare-ups. Further, some patients cannot benefit from this treatment due to the presence of other conditions such as allergies, hypertension, nephropathies, etc. Hence, there is a need to provide an improved treatment for acute inflammation, especially joint inflammation.

Specialized pro-resolving lipid mediator (SPM, also termed specialized pro-resolving mediators) belong to a large and growing class of cell signaling molecules formed in cells by the metabolism of polyunsaturated fatty acids (PUFA) by one or a combination of lipoxygenases, cyclooxygenase, and cytochrome P450 monooxygenase enzymes. SPM are key players in orchestrating the natural resolution of inflammation. These molecules include maresins (maresin-1 and maresin-2), D-series resolvins (resolvin D1, D2 D3 or D4), E- series resolvins (resolvin E1 or E2), protectins (protectin D1 or neuroprotection D1) and lipoxins (lipoxin A4). Lipoxins are derived from arachidonic acid, E- series resolvins are derived from the long-chain n-3 fatty acid eicosapentaenoic acid (EPA) and D-series resolvins, protectins/neuroprotectins and maresins, are all derived from the n-3 fatty acid docosahexaenoic acid (DHA).

The present invention provides a treatment for acute inflammation based on the combination of two of those SPMs, particularly Lipoxin A4 and Maresin-1.

### DESCRIPTION OF THE FIGURES

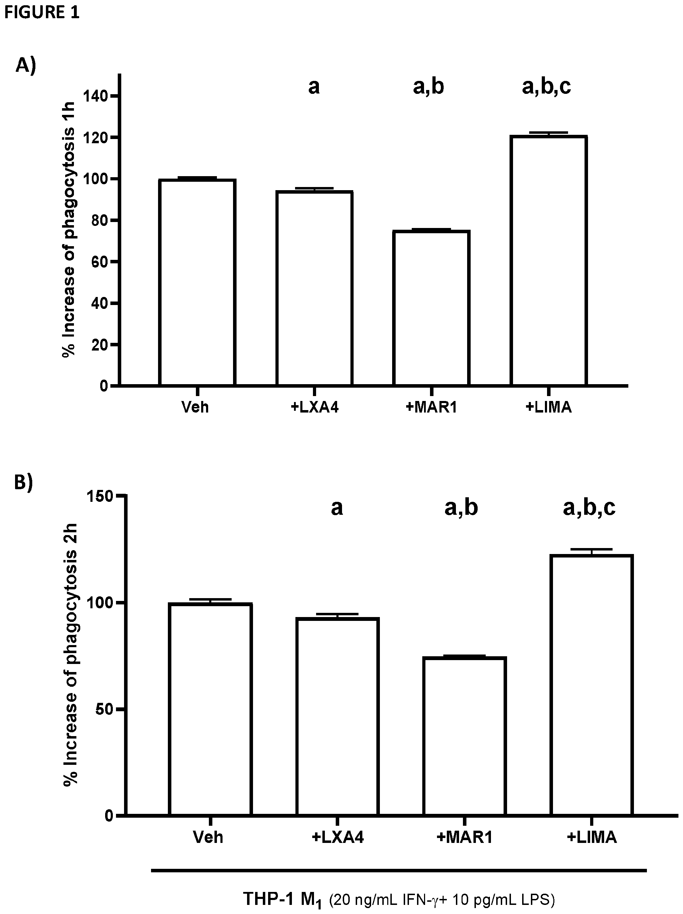

**Figure 1****.** Assessment of the phagocytic capacity of THP1 M1 macrophages one **(A)** and two **(B)** hours after the addition of zymosan fluorescent particles and treatments. All THP1 cells were pre-treated with IFN-Y (20 ng/mL) and LPS (10 pg/mL) to promote differentiation to phagocytic M1 phenotype. Groups correspond to: Veh = untreated vehicle; +LXA4= cells treated with 10 nM Lipoxin A4; +MAR1= cells treated with 10 nM Maresin-1; +LIMA= cells treated with the combination of 10 nM Lipoxin A4 and 10 nM Maresin-1. The result is expressed as the percentage of fluorescence increase emitted at a certain time. Bars show the mean and SEM. Statistical significance is considered p<0.05 (*n*=*5*): a=significant vs control V, b=significant vs LXA4, c=significant vs MAR1.

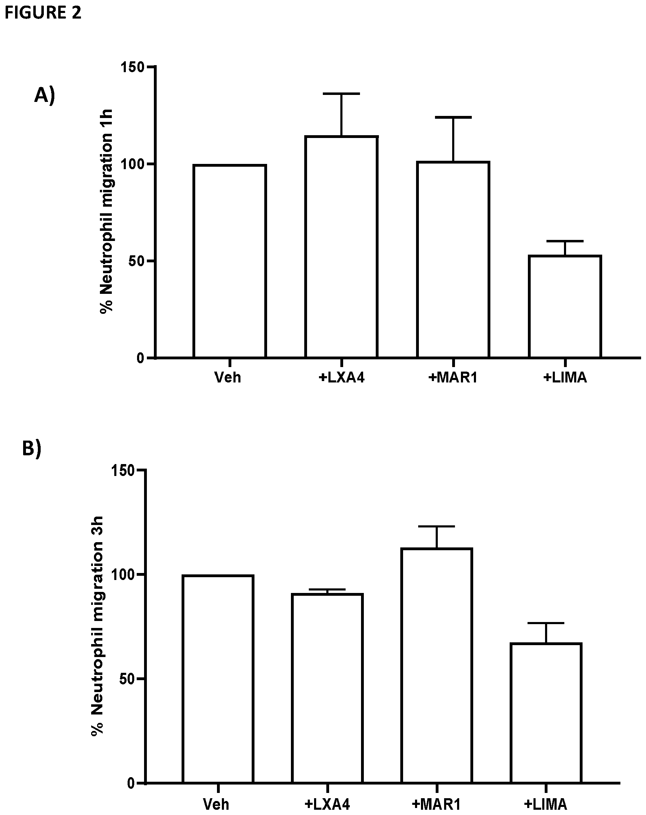

**Figure 2****.** Inhibitory percentage assessment of human neutrophil migration one **(A)** and three **(B)** hours after addition of a chemotactic stimulus (IL-8 20 nM+ HSA 0.5%). Neutrophils were pre-treated with vehicle or SPMs 45 minutes before their addition over the chemotactic stimulus. Groups correspond to: Veh = untreated vehicle; +LXA4= cells treated with 1 nM Lipoxin A4; +MAR1= cells treated with 1 nM Maresin-1; +LIMA= cells treated with the combination of 1 nM Lipoxin A4 and 1 nM Maresin-1. The result is expressed as the percentage of migrating neutrophils through the 5 µm pore filter. Bars show the mean and SEM. Statistical significance is considered p<0.05 (*n*=*4*): a=significant vs control V, b=significant vs LXA4, c=significant vs MAR1. Each n corresponds to an independent healthy volunteer donor.

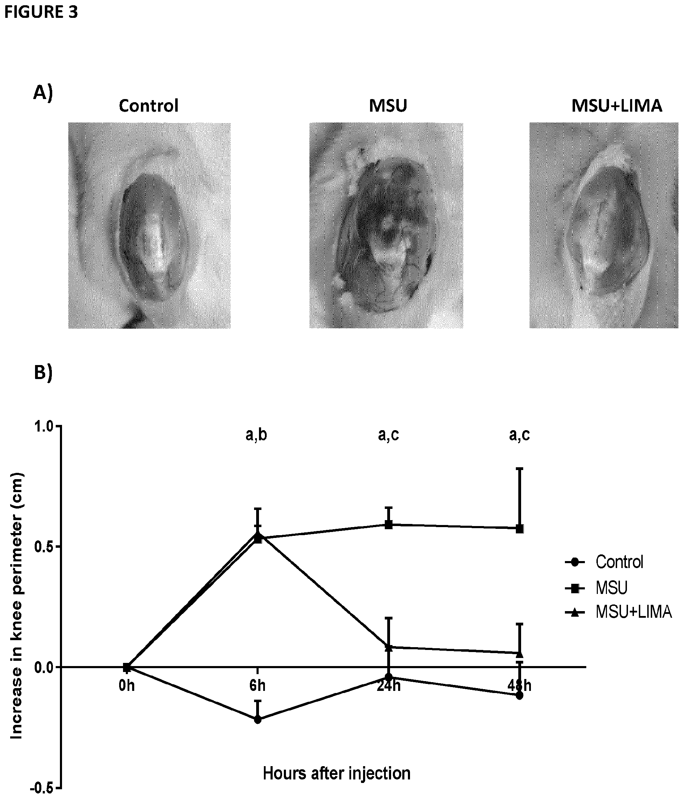

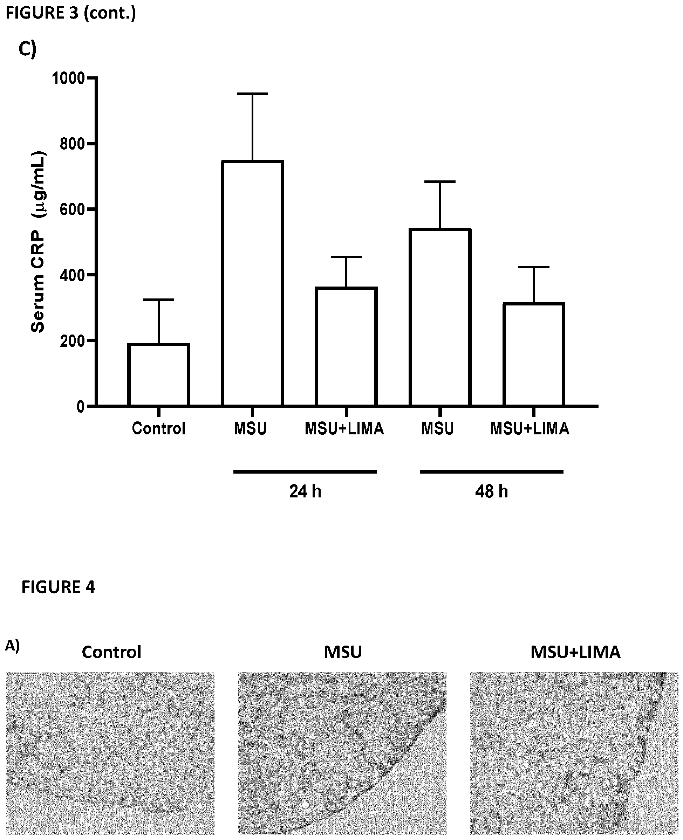

**Figure 3. A)** Representative images of rabbit knee evidencing swelling and haemorrhagic leakage 48 h after MSU injection in Control, MSU and MSU+LIMA groups. **B)** Evolution of joint swelling by increase in knee perimeter in each group of animals along the study (*n*=*6* joints/group). **C)** Serum C-Reactive protein concentration levels at 24h and 48h after MSU intraarticular injections (*n*=3/group). Bars show the mean and SEM. Statistical significance is considered p<0.05: a=MSU vs Control, b=MSU+LIMA vs Control, c=MSU vs MSU+LIMA. MSU: monosodium urate crystals; LIMA: Lipoxin A4+ Maresin-1; CRP: C-Reactive Protein.

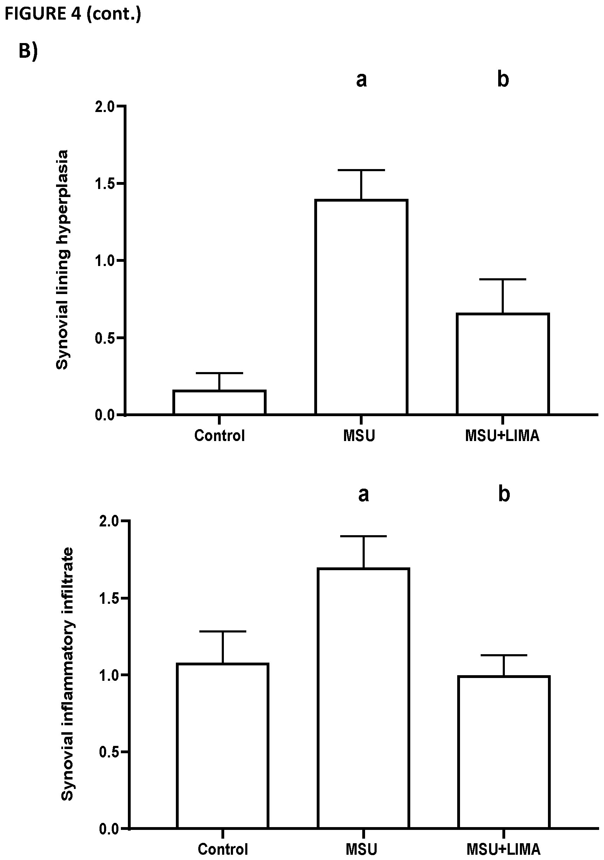

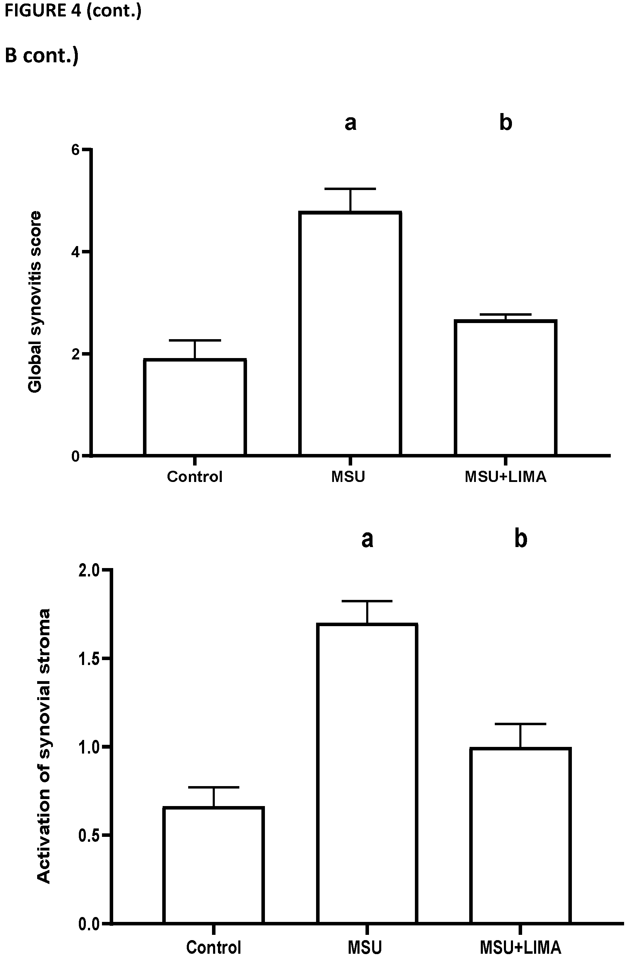

**Figure 4. A)** Representative sections of hematoxylin-eosin staining from the synovium 48 hours after MSU intraarticular injections in Control, MSU and MSU+LIMA groups. Objective magnification: 20 X. **B)** Synovitis histological evaluation according to Krenn guidelines in terms of lining hyperplasia, inflammatory infiltrate, activation of stroma and global score. Bars show the mean and SEM. Statistical significance is considered p<0.05 (*n*=6 joints/group): a=MSU vs Control, b=MSU vs MSU+LIMA. MSU: monosodium urate crystals; LIMA: Lipoxin A4+ Maresin-1.

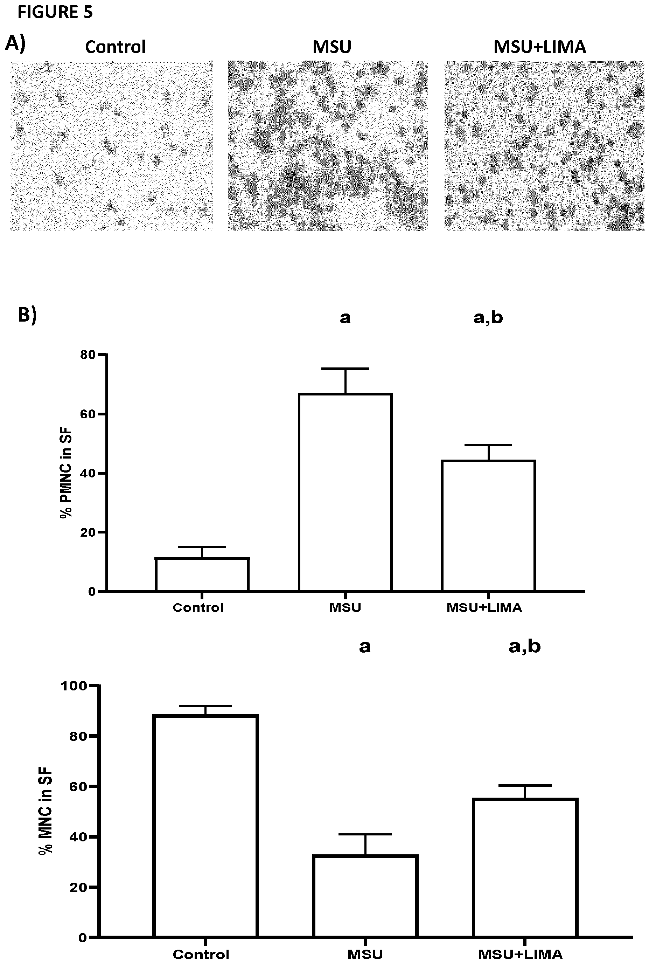

**Figure 5. A)** Representative images of synovial fluid samples of Control, MSU and MSU+LIMA groups with differential panoptic staining at 48h after MSU intraarticular injection. Objective magnification: 40 X. **B)** Distribution of PMNC and MNC at 48h of Control, MSU and MSU+LIMA groups. Bars show the mean and SEM. Statistical significance is considered p<0.05 (*n*=*6*/group): a=vs Control, b=vs MSU. MSU: monosodium urate crystals; LIMA: Lipoxin A4+ Maresin-1; PMNC: polymorphonuclear cell; MNC: mononuclear cell.

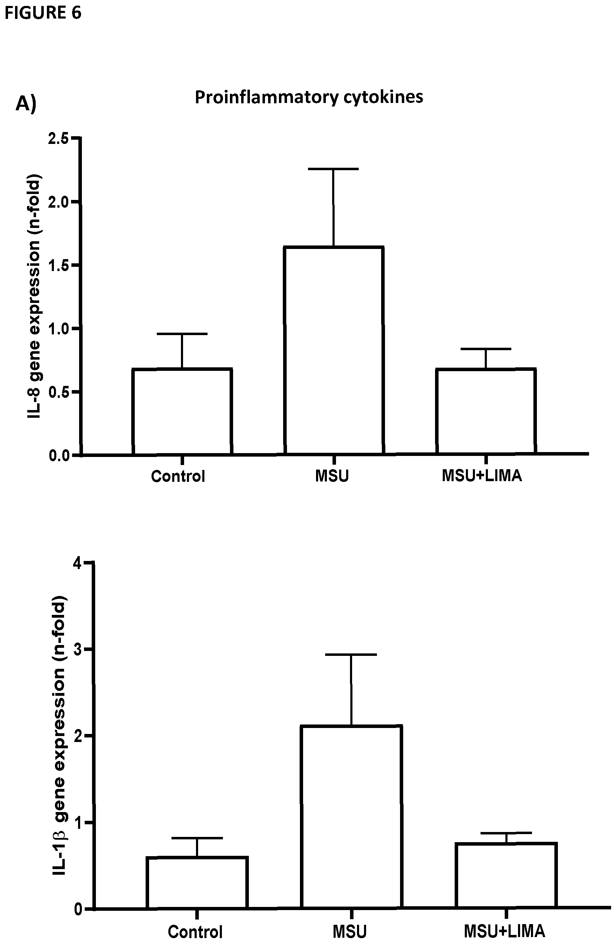

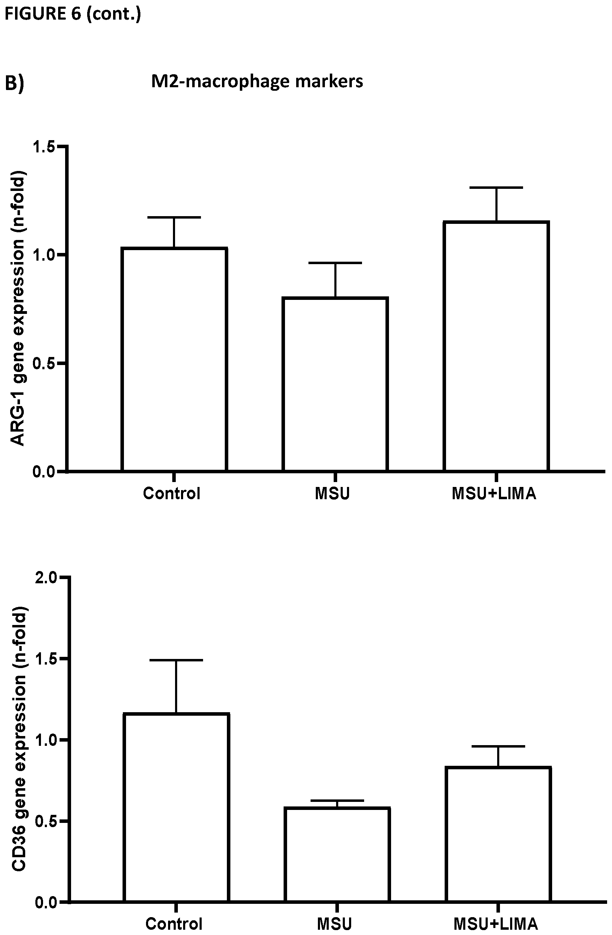

**Figure 6****.** Synovial gene expression of proinflammatory cytokines **(A)** and M2 polarization markers **(B)** in Control, MSU and MSU+LIMA groups. Bars show the mean and SEM. Statistical significance is considered p<0.05 (*n*=*3*/group). MSU: monosodium urate crystals; LIMA: Lipoxin A4+ Maresin-1.

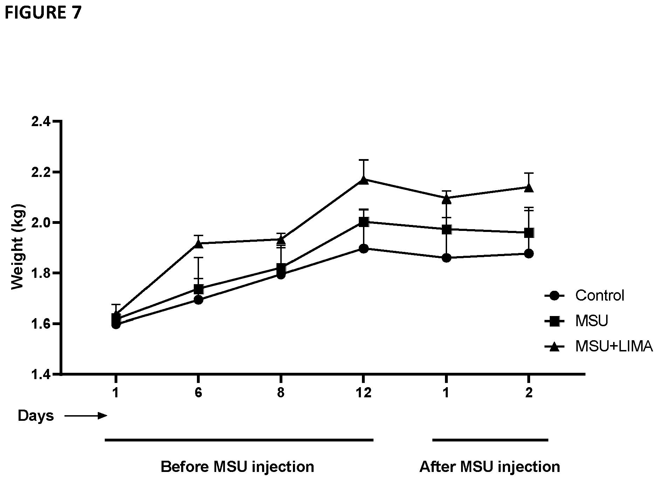

**Figure 7****.** Weight expressed in kg along the days of study before and after MSU injection for Control, MSU and MSU+LIMA groups. Bars show the mean and SEM. Statistical significance is considered p<0.05 (n=3/group). MSU: monosodium urate crystals; LIMA: Lipoxin A4+ Maresin-1.

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the term "about" (or "around") means the indicated value ± 1 % of its value, or the term "about" means the indicated value ± 2 % of its value, or the term "about" means the indicated value ± 5 % of its value, the term "about" means the indicated value ± 10 % of its value, or the term "about" means the indicated value ± 20 % of its value, or the term "about" means the indicated value ± 30 % of its value; preferably the term "about" means exactly the indicated value (± 0 %).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred. When used herein "consisting of" excludes any element, step, or ingredient not specified in the claim element.

The term "condition" (as in medical condition) as used herein is intended to be generally synonymous, and is used interchangeably with, the terms "pathology", "disorder" and "disease", in that all reflect an abnormal condition of the body or of one of its parts that impairs normal functioning and is typically manifested by distinguishing signs and symptoms.

A "therapeutically effective amount" as referred herein is defined as an amount of a compound that, when administered to a subject, is sufficient to effect such treatment of a conditioned state. The "therapeutically effective amount" may vary depending on the compound, and condition being treated, the age and relative health of the subject, the route and form of administration, the judgment of the one having ordinary skill in the art, and other factors.

The term "treatment" as used herein refers to the medical care given to a patient for a disease or injury. This term includes curing the disease but also ameliorating, mitigating, or reducing the symptoms of said disease. Thus, the term "therapeutic treatment" or "treatment" as used herein refers to bringing a body from a pathological state or disease back to its normal, healthy state. The term "therapeutic treatment" or "treatment" as used herein, also refers to the mitigation, amelioration, or reduction of the harmful effect of radiation in a subject, or in some manner reducing the symptoms associated with such effects. The term "prophylactic treatment" as used herein refers to preventing a pathological state.

A "Specialized pro-resolving lipid mediator" (SPM, also termed specialized pro-resolving mediators) is a large and growing class of cell signalling molecules formed in cells by the metabolism of polyunsaturated fatty acids (PUFA) by one or a combination of lipoxygenase, cyclooxygenase, and cytochrome P450 monooxygenase enzymes. It has been shown that SPM are key players in orchestrating the resolution of inflammation. These molecules include maresins (maresin-1 and maresin-2), D-series resolvins (resolvin D1 , D2 D3 or D4), E- series resolvins (resolvin E1 or E2, protectins (protectin D1 or neuroprotection D1) and lipoxins (lipoxin A4 or aspirin-triggered lipoxin). Lipoxins are derived from arachidonic acid, E- series resolvins are derived from the long-chain n-3 fatty acid eicosapentaenoic acid (EPA) and D-series resolvins, protectins/neuroprotectins and maresins, are all derived from the n-3 fatty acid docosahexaenoic acid (DHA).

The term "kit of parts" as used herein refers to a combined preparation wherein the active ingredients are physically separated for use in a combined therapy by simultaneous administration or sequential administration to the patient.

### DETAILED DESCRIPTION

The immune response comprises not only pro-inflammatory and anti-inflammatory pathways but also pro-resolution mechanisms that serve to balance the need of the host to defend from injuries while preventing excess inflammation and bystander tissue damage. Two fundamental processes in resolving acute inflammation are the promotion of phagocytosis/efferocytosis and the inhibition of chemotaxis. The phagocytosis/efferocytosis involves the removal of apoptotic cells, while the inhibition of chemotaxis stops the migration of white cells, mainly neutrophils, to the injured area. The deregulation of said processes results in a severe immune system malfunction and loss of homeostasis, which is associated with serious diseases.

Lipoxin A4 (LXA4) and Maresin-1 (MAR1) are two members of the SPM family. The authors of the present invention investigated their ability as pro-resolving mediators in the context of acute inflammation. It was surprisingly found that, when LXA4 and MAR1 are administered alone, they are not strong inflammation-revolving mediators, since they have poor pro-efferocytic effect on THP-1 macrophages (see Example 1) and are not able to inhibit neutrophil migration at early times (see Example 2). However, when they are administrated together in a combined treatment, they provide an unexpected and advantageous synergistic effect on the inflammation observed in the synovium that goes beyond their effect when administrated separately. This synergistic effect results in an effective macrophage phagocytic activity (see Example 1), and a strong inhibition of neutrophil migration, especially at early times (see Example 2). These results were validated *in vivo,* supporting the potential use of the combined treatment (LXA4 + MAR1, also called LIMA treatment) to reduce systemic and acute inflammation (see Example 3), to prevent histopathological lesions in the synovial membrane of inflamed joints (see Examples 4), to inhibit neutrophilic migration (see Example 5), and to reduce proinflammatory gene expression and stimulate pro-resolving pathway (see Example 6). Further, the *in vivo* results also showed that this treatment is not only biocompatible (it did not alter the joint tissue nor affected the weight of the treated animals) but allowed joint recovery when the inflammation resolved in treated animals (see Example 7).

In view of this, **a first aspect** of the present invention relates to a composition, also called the composition of the invention, that comprises at least two members of the SPM family, preferably at least a Lipoxin and a Maresin. In an embodiment, the Lipoxin is Lipoxin A4. In an embodiment, the Maresin is Maresin-1. Thus, a preferred embodiment of the first aspect refers to a composition comprising at least Lipoxin A4 and Maresin-1. Preferably, the only SPM mediators that the composition comprises are Lipoxin A4 and Maresin-1. Preferably, the composition comprises isolated Lipoxin A4 and Maresin-1.

In an embodiment, the compounds present in the composition, preferably Lipoxin A4 and Maresin-1, are autologous, allogenic, or xenogenic. As used herein "autologous" is understood as referring to a preparation where said compounds are obtained from a donor, and wherein the donor and the recipient (the one receiving the composition) are the same individual. As used herein "allogeneic" or "xenogenic" is understood as referring to a preparation where said compounds are obtained from a donor and wherein the donor and the recipient are not the same individual. In an embodiment, the compounds present in the composition, preferably Lipoxin A4 and Maresin-1, are artificially synthetised, i.e., they are not obtained from an individual.

Lipoxin A4 (from hereinafter "LXA4")) is 5S,6R, 15S-irihydroxy-eicosa-7E,9E, 1 Z, 13E-tetraenoic acid). In a prefer embodiment, the LXA4 is in the form of LXA4 hydrate, salt, solvate, hydrated salt, or its optical isomer, racemate, diastereoisomer or enantiomer thereof. In another embodiment, the LXA4 is a LXA4 derivative, an analogue, a mimetic, or or a functional equivalent thereof. By "mimetic" is referred herein as to a compound that possesses the same activity as the compound referred to in the specific embodiment. By "analogue" is referred herein to compounds that are similar in structure and/or function to the compound referred to in the specific embodiment. By "functional equivalent" is referred herein to compounds that differ in their structure from that of the compound referred to in the specific embodiment, but which perform the same function and provide the same utility or technical effect as the compound referred to in the specific embodiment. For instance, a functional equivalent of LXA4 is a compound that differs in its structure from LXA4, but that has the same effect as LXA4, especially the same synergistic effect of LXA4 when combined with MAR1.

Preferably, the LXA4 is a stable analogue. By "stable" is referred herein as to a compound that performs the same function and provide the same utility of technical effect as LXA4 but has an improved or longer half-lives in vitro and in vivo than native LXA4. In a preferred embodiment, the LXA4 stable analogues comprise bulky substitutions on carbon 15, or at the carbon 20 end of the molecule, as described in Maddox et al. 1997 (doi: https://doi.org/10.1074/jbc.272.11.6972). Most preferably, the LXA4 stable analogue is selected from the list of 15(R/S)-methyl-LXA4, 16-phenoxy-LXA4, or their methyl esters thereof, as represented by the following formulas:

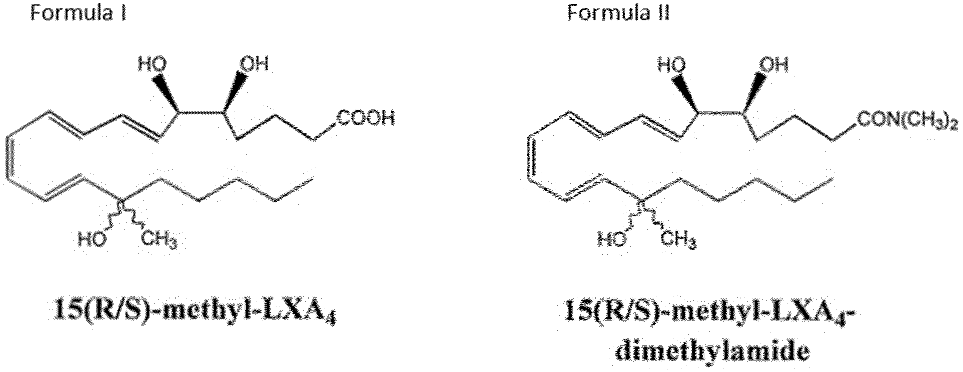

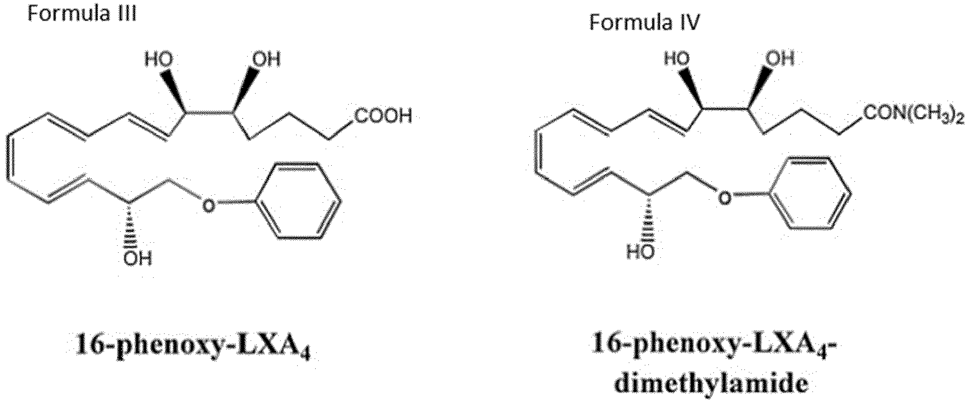

Maresin-1 (from hereinafter "MAR1") is a 13R,14S-dihydroxy DHA formed by recombinant human macrophage 12-lipoxygenase and soluble epoxide hydrolase co-incubated with DHA. In a prefer embodiment, the MAR1 is in the form of MAR1 hydrate, salt, hydrated salt, or its optical isomer, racemate, diastereoisomer or enantiomer thereof. In another embodiment, the MAR1 is a MAR1 derivative, an analogue, a mimetic, or a functional equivalent thereof.

In a preferred embodiment, the LXA4 and MAR1 compounds comprised in the composition of the invention are modified to provide a stable composition. As used herein, a "stable composition" may refer to a formulation in which the active ingredient (i.e., the LXA4 and MAR1), essentially retains its physical stability and/or chemical stability and/or biological activity upon storage and/or upon administration. In an embodiment, the LXA4 and MAR1 are modified to make them resistant to oxidation reactions, such as the ones represented in Formulas I, II, III IV above.

In a preferred embodiment, the composition of the first aspect is formulated as a cosmetic composition, pharmaceutical composition, food formula, food ingredient or supplement, functional food, nutritional supplement, nutraceutical composition or is in the extract of a natural product or biological sample.

In a more preferred embodiment, the composition is a pharmaceutical composition. In an embodiment, the composition is a pharmaceutical composition, and it further comprises one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like. "Pharmaceutically acceptable salt" as used herein means that the salt derived from the corresponding compound is suitable for administration to a subject to achieve the treatments described herein, without unduly deleterious side effects in light of the severity of the disease and necessity of the treatment. The pharmaceutically acceptable acid addition salts can conveniently be obtained by treating the base form with such appropriate acid. However, salts of acids and bases which are non-pharmaceutically acceptable may also find use, for example, in the preparation or purification of a pharmaceutically acceptable compound. All salts, whether pharmaceutically acceptable or not are included within the scope of the present invention. Preferably, the pharmaceutically acceptable acid and base addition salts as mentioned herein are meant to comprise the therapeutically active non-toxic acid and base addition salt forms which the compounds as disclosed herein are able to form.

Dose ranges of the pharmaceutical compositions can be adjusted as necessary for the treatment of individual patients and according to the specific condition treated. Any of a number of suitable pharmaceutical formulations may be utilized as a vehicle for the administration of the composition of the present invention and maybe a variety of administration routes are available. The particular mode selected will depend of course, upon the particular formulation selected, the severity of the disease, disorder, or condition being treated, and the dosage required for therapeutic efficacy.

In a more preferred embodiment, said composition is a nutraceutical and/or a food composition. For the purposes of the present invention the term nutraceutical means a "food-drug", i.e. a health food that associates nutritional components with the curative properties of natural active ingredients of proven and recognised effectiveness, in this case LXA4 and MAR1. A composition of a "food" or "food ingredient or supplement", "functional food" or "nutritional supplement" as described above may in principle take any form suited for consumption by human or animal. Nutraceuticals can also be defined as natural products that are used to supplement the diet by increasing the total dietary intake of important nutrients. This definition includes nutritional supplements such as vitamins, minerals, herbal extracts, antioxidants, amino acids, and protein supplements. A "nutraceutical composition" is defined herein as a food composition fortified with ingredients capable of producing health benefits. Such a composition in the context of the present invention may also be indicated as foods for special dietary use; medical foods; and dietary supplements. For example, the food item or supplement may help to prevent or reduce symptoms associated with an inflammatory condition and the like.

The composition of the invention can be a food composition. The food composition can be a liquid or a solid composition. The composition may be further used in the preparation of functional food or food that have an additional function upon addition of LXA4 in combination with MAR1, as defined herein. Nutritional, dietary or nutraceutical compositions or food supplements or functional food can be found in different presentations, such as pastilles, pills, tablets, capsules, soft gelatin capsules, gelatin capsules, wafers, effervescent tablets, liquids (solution, suspension, syrup), granules and powders, all of which are included as particular embodiments within the scope of the present invention. Dietetically acceptable excipients are known for the skilled person for obtaining any of the preceding formulations, and they are included within the scope of the present invention.

As with the pharmaceutical composition, the amount of active ingredient in the nutraceutical or food composition will depend on several factors. The nutraceutical food product will generally comprise a concentration that is sufficient to provide a consumer with an effective amount of active ingredient upon consumption of a regular (e.g. daily) portion of the food product.

In addition, the composition comprising LXA4 and MAR1 might contain other ingredients. For example, the composition may be mixed, dissolved, emulsified (e.g., in oil/water, water/oil, or double emulsions), or suspended in a matrix or base. The matrix or base can, e.g., be an edible oil such as ω-3 PUFA- containing oils, a ω-3 PUFA concentrate containing high levels of EPA, or DELA, or mixtures of EPA and DELA, or another edible oil suitable for consumption or administration. The matrix or base might also be water or an aqueous buffer.

To enhance shelf life, the composition might also contain one or more stabilizers including antioxidants such as one or several tocopherols, ascorbic acid and ascorbyl-fatty acid derivatives, and other antioxidants which are commonly used in the stabilization of dietary oils. The compositions might also include one or more active ingredients such as aspirin, other non-steroidal anti-inflammatory drugs, vitamins, anti-oxidants, flavonoids, minerals, trace elements, fatty acids, lycopene, S-adenosylmethionine, oleocanthal, resveratrol, pterostilbene, bioactive proteins and/or peptides. Specific nutritional supplements can be made to support specific health conditions that include a fish oil, a krill oil, or a long-chain ω-3 PUFA concentrate supplemented with the composition of the invention.

Different options regarding pharmaceutical formulations, structural modifications, and delivery systems, can help to facilitate the administration of LXA4 and MAR1 and are also included herein. Some examples include prodrugs (carboxyesters, sulfonates, sulfates, phosphates, acetals, or carbamates or carbonates), nanoemulsions, polyionic/polymeric shells encapsulating micro or nanoparticles, liposomes, or exosomes. In particular, any pharmaceutically acceptable formulation of LXA4 and MAR1 are preferred.

Preferably, the composition of the present invention is in a form suitable for any type of administration including, but not limited to, parenteral, intraarticular, oral or topic administration.

In **a second aspect,** the composition is used in therapy or as a medicament. Preferably, the use is in the treatment, prevention, amelioration or reduction of inflammation or diseases that occur with inflammation. With "prevention" is meant keeping the disease from happening. With "amelioration" is meant an improvement in a patient's disease, or the activity of making an effort to correct, or at least make more acceptable said disease.

With "reduction" or "mitigation" is meant decreasing the severity, seriousness or painfulness of the disease. "Inflammation" refers to the biological response elicited against harmful stimuli, such as, but not limited to, the presence of damaged cells, microcrystals, or irritants. Inflammation is a protective attempt by the organism to remove the injurious stimuli as well as initiate the healing process for the tissue. Accordingly, the term "inflammation" includes any cellular process that leads to the production of pro-inflammatory cytokines, inflammation mediators and/or the related downstream cellular events resulting from the actions of the cytokines thus produced, for example, fever, fluid accumulation, swelling, abscess formation, and cell death. Pro-inflammatory cytokines and inflammation mediators include, but are not limited to, IL-1-alpha, IL-1-beta, IL-6, IL-8, IL-11, IL-12, IL-17, IL-18, TNF-alpha, leukocyte inhibitory factor (LIF), IFN-gamma, Oncostatin M (OSM), ciliary neurotrophic factor (CNTF), TGF-beta, granulocyte-macrophage colony stimulating factor (GM-CSF), and chemokines that chemoattract inflammatory cells. Inflammation can include both acute responses (i.e., responses in which the inflammatory processes are active) and chronic responses (i.e., responses marked by slow progression and formation of new connective tissue).

Preferably, the composition of the invention is used to in the treatment, prevention, amelioration or reduction of acute inflammation or diseases that occur with acute inflammation, preferably local inflammation. By "acute inflammation" is referred to the presence of at least one symptom selected from a) white blood cell count (WBC) > about 12,000 µ/L or < about 4000µ/L, b) body temperature > about 38°C or < about 36°C, c) heart rate > about 90 beats/minute, d) a respiratory rate > about 20 breaths/minute or a partial pressure of CO2 of less than about 32 mm Hg, and/or e) more than 10% immature white blood cells among the counted white blood cells. Preferably, a situation of acute inflammation is associated to fever (hyperthermia), i.e. a body temperature higher than about 38°C. In some embodiments, the acute inflammation is a systemic acute inflammation. However, more preferably, the acute inflammation is a local acute inflammation. Preferably, the local acute inflammation is an inflammation that occurs in a localized area of the body, such as the joint, in response to damage-associated molecular patterns ("DAMPs"). DAMPs include, but are not limited to, a harmful or offending agent (such as a trauma, toxins, or accumulation of microcrystals).

Preferably, the local acute inflammation treated with the composition of the invention is caused by an acute joint pathology, is located in one or more joints, and involves one or more of: an increase in joint perimeter, neoangiogenesis, systemic increase in CRP and TNF; a decrease in the production of anti-inflammatory molecules such as IL-10; a decrease in the local synthesis of SPMs in the synovial membrane; the activation of the NLRP3 inflammasome; and/or the presence of proinflammatory M1 macrophages in the synovium.

Preferably, the local acute inflammation is a self-limited inflammation. By "self-limiting inflammation" is referred herein to an inflammation that leads to the complete resolution of inflammatory infiltrates and clearance or cellular debris so that the harmed tissue returns to homeostasis. In particular, the composition of the invention is used to reduce the time in which the inflammation, preferably local acute inflammation in one or more joints, is resolved. In a most preferred embodiment, the composition of the first aspect is used in the treatment, prevention, amelioration or reduction of local inflammation or diseases that occur with local inflammation, preferably local self-limiting inflammation, most preferably local, acute, self-limiting inflammation.

In an embodiment, the composition of the invention is used to treat an inflammatory condition in a subject in need thereof. An inflammatory condition is any disease state characterized by inflammatory tissues (for example, infiltrates of leukocytes such as lymphocytes, neutrophils, macrophages, eosinophils, mast cells, basophils and dendritic cells) which provoke or contribute to the abnormal clinical and histological characteristics of the disease state. Inflammatory conditions include, but are not limited to, inflammatory conditions of the skin, inflammatory conditions of the lung, inflammatory conditions of the joints, inflammatory conditions of the gut, inflammatory conditions of the eye, inflammatory conditions of the endocrine system, inflammatory conditions of the cardiovascular system, inflammatory conditions of the kidneys, inflammatory conditions of the liver, inflammatory conditions of the central nervous system, or sepsis-associated conditions, in some embodiments, the inflammatory condition is associated with wound healing. Inflammation is often part of the wound and/or tissue healing process and the methods and compositions described herein can be used to enhance or promote wound and/or tissue and/or joint healing.

In a prefer embodiment, the inflammatory condition is an inflammatory condition of the joints. Preferably, the condition treated with the composition of the invention is a joint disorder characterized by accumulation of microcrystals (such as the accumulation of monosodium urate (MSU), calcium pyrophosphate (CPP) and basic calcium phosphate (BCP), oxalate and/or cholesterol) in one or more joints, preferably wherein the accumulation of said microcrystals causes or will cause inflammation, preferably local acute inflammation.

Preferably, the condition treated with the composition of the invention is a condition selected from the group consisting of musculoskeletal diseases including gout, pseudogout, arthritis, arthritis associated with pyrophosphate deposits or other microcrystal arthropathies, traumatic arthritis, reactive arthritis, osteoarthritis, tendinitis, tenosynovitis, and rheumatoid arthritis. Most preferably, the condition treated is a joint disorder characterized by accumulation of crystals, preferably microcrystal arthropathy, most preferably gout, pseudogout, and/or chondrocalcinosis.

In an embodiment, the composition of the first aspect is administrated in a subject in need thereof. A "subject in need" of treatment for a particular condition can be a subject having that condition, diagnosed as having that condition, or at risk of developing that condition. Preferably, the subject is a mammal. The mammal can be a human, non-human primate, mouse, rat, dog, cat, horse, or cow, but are not limited to these examples. Mammals other than humans can be advantageously used, for example, as subjects that represent animal models of, for example, inflammation or a disease that causes inflammation. In addition, the methods described herein can be used to treat domesticated animals and/or pets.

A subject can be male or female, preferably human male or female, of any age. Subject can be one who has been previously diagnosed with or identified as suffering from or having a condition in need of treatment (e.g. inflammation, acute inflammation, joint inflammation or microcrystal accumulation) or one or more complications related to such a condition, and optionally, but need not have already undergone treatment for a condition or the one or more complications related to the condition. Preferably, the subject to be treated is a subject having inflammation or a disease that causes inflammation, preferably a subject having acute inflammation, most preferably a subject having joint acute inflammation. Also preferably, the subject to be treated has been diagnosed with one or more of musculoskeletal diseases including gout, pseudogout, arthritis, arthritis associated with pyrophosphate deposits or other microcrystal arthropathies, traumatic arthritis, reactive arthritis, osteoarthritis, tendinitis, tenosynovitis, and rheumatoid arthritis. Most preferably, the subject in need of the treatment is suffering from, or has been diagnosed with, microcrystal arthropathy, a rheumatoid disease, or arthritis, most preferably gout, pseudogout and/or chondrocalcinosis.

Alternatively, a subject can also be one who has not been previously diagnosed as having a condition in need of treatment or one or more complications related to such a condition. For example, a subject can be one who exhibits one or more risk factors for a condition, or one or more complications related to a condition or a subject who does not exhibit risk factors, preferably the condition is any of the above mentioned conditions.

The composition of the invention can be administered by any appropriate route which results in an effective treatment in the subject in need thereof, including, but not limited to, orally or parenteral routes, including intravenous, intraarterial, intrathecal, intramuscular, by injection, transdermally, topical, by direct application (e.g. to the skin or joint or to tissue exposed by, for example, surgery or injury), subcutaneous, transdermal, airway, intraarticular, intrathecal, nasal, rectal. Preferably, the administration is topical on the site of inflammation, such as on the join. Preferably, the composition is administered intraarticularly. Preferably, the composition is administered intraarticularly when the subject is suffering from, or has been diagnosed with, crystal arthropathy or arthritis, most preferably gout, pseudogout and/or chondrocalcinosis.

In an embodiment, the administration of the compounds comprised in the composition of the first aspect, preferably LXA4 and MAR1, can be simultaneous (at the same time) or sequential. "Sequential administration" as used herein refers to the administration of LXA4 or a composition comprising thereof shortly before or after administration of MAR1 or a composition comprising thereof. "Shortly before or after" as used herein is understood as within an interval of 1 to 24 hours, preferably 1 to 12 hours, preferably 1 to 3 hours, preferably within 2 hours, more preferably within 1 hour or less, such as within 45 minutes, 30 minutes, 15 minutes or less. In an embodiment, MAR1 is administered first followed by LXA4. In another embodiment, LXA4 is administered first followed by MAR1. In a preferred embodiment, LXA4 and MAR1 administration is carried out simultaneously. In an embodiment, the LXA4 and MAR1 are administered in combination simultaneously, as a part of a single composition or as part of two different compositions.

The administration can be carried out only once, or there can be more than one administration. In a preferred embodiment, the compounds comprised in the composition of the first aspect are administered several times. In a particular embodiment, the administration may comprise at least one, two, three, four, five, six, seven or more doses.

The administration can be performed before, during or after the condition to be treated, prevented, ameliorated o reduced. For example, the administration can be performed at least 10, 9, 8, 7, 6, 5, 4, 3, 2, 1 days before or after the condition, preferably inflammation, occurs or is diagnosed. In an embodiment, the administration carried out 1, 2, 3, 4, 5, 6, 7, 8, 9, 10 or more than 10 days, such as 20 or 30 days, or more than one moth, such as 2, 3, 5, 6, 8, 9 months after diagnosis or the manifestation of the condition. In a particular embodiment, the administration is carried out on the same day where the condition, such as inflammation, occurs, either before or after it. In an embodiment, the administration consists of several doses administered during several days before, on the same day, and/or after the condition, such as inflammation, occurs. In a preferred embodiment, LXA4 and MAR1, or any pharmaceutically acceptable salts or derivatives or functional equivalents thereof, are administrated several times simultaneously or sequentially before, during and/or after the condition. In a preferred embodiment, the composition of the invention is administered at early times after the production of inflammation, such as 1, 2, 3, 4, 5, 6, 7, 8, 9, 10, 12, 14, 16, 18, 20, 22, or 24h after the generation of inflammation. Most preferably, the composition of the invention is administered as soon as possible after the production of inflammation, preferably within the next 3-5 days after the appearance of the symptoms of inflammation.

The optimal dose will be selected according to the administration route, treatment regime and/or administration schedule, and the condition, having regard to the existing toxicity and effectiveness data. In a preferred embodiment, the composition for use according to the second aspect of the present invention is administered in a dosage capable of providing a pro-resolution of inflammation effect. By "pro-resolution of inflammation effect" is understood the ability to reduce the inflammation, preferably local inflammation, preferably including one or more of: reducing or stopping neutrophil infiltration, reducing the concentration of pro-inflammatory cytokines (such as IL-10, TGFβ, etc), promoting the synthesis of anti-inflammatory cytokines and the release of growth factors that allow tissue reconstruction inducing the healing process and the recovery of the inflamed tissue, stimulating non-phlogistic recruitment of monocytes, stimulating macrophage polarization to an anti-inflammatory phenotype (M2 phenotype), enhancing macrophage phagocytosis of apoptotic neutrophils, increasing the exit of phagocytes from the inflammatory site to the lymphatics; and/or stimulating mucosal defence and the removal of cell debris.

Preferably, the composition is administered in a dosage capable of inhibiting neutrophilic chemotaxis and/or promoting macrophage-mediated phagocytosis in the inflamed tissue or place, resulting in the acceleration of the resolution of the inflammation. Accordingly, in the present invention, the dosage of LXA4 and MAR1 is not particularly restricted.

In a **third aspect,** the present invention relates to the use of the composition of the invention for one or more of the following purposes:
- to reduce the concentration of pro-inflammatory cytokines (such as IL-10, TGFβ) in an inflamed tissue,
- to increase or promote the synthesis of anti-inflammatory cytokines and the release of growth factors that allow tissue reconstruction in an inflamed tissue,
- to increase or promote the healing process and the recovery of the inflamed tissue,
- to increase non-phlogistic recruitment of monocytes to an inflamed tissue,
- to increase or stimulate macrophage polarization to an anti-inflammatory phenotype (M2 phenotype) in an inflamed tissue,
- to increase or enhance macrophage phagocytosis of apoptotic neutrophils in an inflamed tissue,
- to increase the exit of phagocytes from the inflammatory site to the lymphatics, and/or
- to decrease or remove of cell debris in an inflamed tissue.

The increase/decrease of those parameters are preferably measured as compared to an untreated patient, which is a control patient. They can be measured by any suitable technique known in the art, for example those explained in the Examples provided below. Preferably, the untreated patient is a patient suffering from acute local inflammation, preferably suffering from one or more musculoskeletal diseases including gout, arthritis, arthritis associated with pyrophosphate deposits or other microcrystal arthropathies, traumatic arthritis, reactive arthritis, osteoarthritis, tendinitis, tenosynovitis, and rheumatoid arthritis. Most preferably, the untreated patient is suffering from microcrystal arthropathy or arthritis, most preferably gout, pseudogout and/or chondrocalcinosis.

In an embodiment, the increase/decrease of those parameters are preferably a statistically significant increase/decrease. By "statistically significant increase" or "statistically significant decrease" is referred herein as the determination by an analyst that the increase or decrease, respectively, in any of the above parameters is not explainable by chance alone, and thus it is influenced or caused by the treatment with the composition of the invention. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase or decrease of any of the parameters defined above is statistically significant when a statistical test is performed to compare it to the basal level in a reference or untreated cell or subject, and wherein the resulting p-value of said statistical test is of 0.1 or lower, preferably 0.05, 0.01, 0.001 or lower.

The administration route, dosage, and timing for the use according to the third aspect use is the same as defined above in the second aspect of the invention. Preferably, the composition is administrated at the site of inflammation or to the inflamed tissue, preferably topically or intraarticularly.

In a **fourth aspect,** the present invention further relates to the use of the composition of the invention in the nutraceutical field or as a nutraceutical composition. The administration route, dosage, and timing for the nutraceutical use is the same as defined above in the second aspect of the invention. Preferably, the composition is administrated orally when used as a nutraceutical composition.

In a **fifth aspect,** the present invention relates to a kit of parts comprising one or more recipients comprising the composition of the invention. Preferably, the recipients comprise LXA4 and MAR1, or any salts or derivatives thereof. Such kits may also include components that preserve the compounds of the composition or that protect against their degradation.

Such kits preferably will comprise, in suitable means, distinct containers for each individual reagent or solution. The kit may comprise one or more containers holding the compounds of the composition of the invention, and other agents. Suitable containers for the compositions include, for example, bottles, vials, syringes, and test tubes. Containers can be formed from a variety of materials, including glass or plastic. A container may have a sterile access port (for example, the container may be a vial having a stopper pierceable by a hypodermic injection needle).

The kit can further comprise a container comprising a pharmaceutically acceptable buffer, such as phosphate-buffered saline. It can also contain other materials useful to the end-user, including other pharmaceutically acceptable formulating solutions such as buffers, diluents, filters, needles, and syringes or other delivery devices. The delivery device may be pre-filled with the compositions.

The kit can also comprise a package insert containing written instructions for methods of treating the conditions explained above, preferably inflammation, preferably acute inflammation, preferably crystal arthritis.

The kits of the third aspect are for use as defined in the second aspects of the invention, or any of its embodiments.

### EXAMPLES

### SUMMARY

Previous studies using an animal model of gout treated with mesenchymal stem cells have shown that the administration of these cells decreased the intensity and duration of the flare. Subsequently, more exhaustive analysis appeared to indicate changes in the regulation of the genes related to the modulation of SPMs. It is relevant to point out that, according to our data, conventional therapy with NSAIDs or glucocorticoids could delay the inflammatory flare resolution and the tissue repair. To study whether SPM administration can directly trigger acute inflammation resolution, we performed *in vitro* and *in vivo* experiments using 2 different SPM, Lipoxin A4 (LXA4), Maresin-1 (MAR1) and its combination, LXA4 + MAR1 (LIMA). Surprisingly, *in vitro,* the addition of none of these SPMs separately had enough potential to inhibit neutrophilic chemotaxis or to promote macrophage-mediated phagocytosis, key events in acute inflammation. However, the combined use of both SPM in LIMA was shown to be an effective treatment to promote both processes. We tested these results *in vivo,* using the previously described rabbit model of gouty arthritis. The analysis of knee swelling and synovial membrane (SM) integrity demonstrated that LIMA intraarticular injection improved knee swelling in the first 18h, and reduced pro inflammatory markers in the blood and in the knee. Finally, it also ameliorates SM histological damage probably as consequence of the significant decrease in inflammatory neutrophil infiltration in the knee. In conclusion, our results show, for the first time, that the specific combination of LXA4 + MAR1 provides a synergistic effect ameliorating the arthritic knee flare in rabbits through the induction of an anti-inflammatory and pro-resolving response, highlighting its relevance as possible new anti-rheumatic treatment.

### MATERIAL AND METHODS

### A) In vitro study

### Isolation of Neutrophils

Blood was collected from four healthy donors using green heparinized blood collection tubes. Thirty mL per donor were obtained. Isolation of viable polymorphonuclear cells (PMNCs) from fresh peripheral blood was performed through a density gradient centrifugation, using Ficoll^{®} medium protocol. Briefly, low-density white blood cells, i.e., mononuclear cells (MNCs) and platelets, form a concentrated band at the interface between the upper plasma layer and the Ficoll^{®} medium, called "buffy coat". High-density cells, such as erythrocytes and neutrophils, are collect at the bottom of the density gradient medium. We carefully removed the fluffy upper phase of the blood and the interface leaving lower bottom layer undisturbed. We added 20 mL of HBSS (12549069, Fisher) and 20 mL of Dextran 3% to promote sedimentation. After 20 min centrifugation at 1G, we saved the supernatant, and we lysated the remaining platelets using a lysis buffer (13,6 mL 10X PBS in 600 mL water). Digestion was stopped using the same volume of re-equilibration buffer (108,1 mL 10X PBS in 500 mL water) and HBSS. After last centrifugation step, the pellet of neutrophils were diluted in 10ml of HBSS for migration assays.

### Migration Assay in Neutrophils

P24 permeable Transwell^{®}, with 5 µm pore size, were used for migration assay (Costar 3421). 1.5×10⁶ isolated neutrophils were cultured in the upper chamber with HBSS (-Ca2+, -Mg+2), and treated with 1 nM of Lipoxin A4 (LXA4, 90410-100, Cayman Chemical), Maresin-1 (MAR1, 10878-100, Cayman Chemical), Lipoxin A4+Maresin-1 (LIMA) or the vehicle (EtOH 100%) for 45 min. After 45 min, Transwells^{®} were placed in HBSS supplemented with 0.5% human serum albumin (HSA, 68209-643-01 Octapharma) and containing 20 nM of IL-8 (REF: SRP3098, Merck) in the lower chamber. After 1 and 3 hours, total number of migrated cells was counted in the lower chamber under microscope, 10× magnification. Assay was performed in duplicate.

### Phagocytosis by Macrophages

We evaluated the pro-phagocytic capacity of SPMs by using a well-established line of monocytes THP-1. First, we differentiated 100.000 THP-1 cells/well in a 96 black plate to M1 macrophage stage (THP1 M1). We incubated them in RPMI (15-040-CV, Corning) + 10% fetal bovine serum (FBS) (10270-106, Gibco) and with 100nM of (phorbol-12-myristate-13-acetate) plus PMA (P1585-1MG, Sigma)) for 24h in the incubator at 37° (*Ozleyen A, Yilmaz YB, Turner TB. 2021 Jan*). After that time, we removed the medium, leaving the cells resting for another 24h in RPMI + 10% FBS. After that period, we incubated the cells on RPMI + 20ng/ml of Interferon-y (IFN-y) (RAX1915031, R&D systems.) plus 10pg/ml of LPS (L6529-1MG, Sigma) for another 24h. Then, we removed the medium and we added 100 µl of reconstructed pHrodo to THP1 M1 (P35361, Invitrogen) plus 10 nM of LXA4, MAR1, LIMA or its vehicle per well; we incubated the plate at 37°C for 2h. We measured the phagocytosis capacity of THP1 M1 as the increase in fluorescence based on the acidification of the particles when they are ingested. Using a fluorescent microplate reader, we measured the absorbance (560ex/585em) at 1h and 2h post treatment. Assay was performed in quadruplicates and 4 types of conditions were tested (THP1 M1+LXA4, THP1 M1+MAR1, THP1 M1+LIMA, THP1 M1+Vehicle). Results were corrected by MTT viability test. Briefly, we added fresh MTT diluted in PBS 1X (1:100) to all wells and incubate the plates 1 h at 37°C. We removed the medium and MTT from the wells and dissolved the purple MTT-formazan crystals by adding 200 µL of DMSO to all wells. We incubated it for 30min at 500 rpm shaking. Finally, we measured the absorbance at 570 nm in the microplate reader.

### B) In vivo study.

### Animal model

To evaluate the effect of our drug combination (LIMA) *in vivo,* we used a well-established rabbit model of gouty arthritis as an animal model for acute inflammation (Bermejo I. 2023).

We employed three groups of three-month old New Zealand White male rabbits (2.0 kg body weight, Granja San Bernardo, Spain): **Control** (Healthy animals with intraarticular injection of LIMA vehicle), **MSU** (animals with intraarticular injection of monosodium urate crystals (MSU) + LIMA vehicle) and **MSU+LIMA** (animals with intraarticular injection of MSU 1ml [50mg/ml] + 3.33 µg/kg of LIMA). Animal protocol lasted 48h.

Animal protocol: MSU was intraarticular injected at time 0h. Six hours and 24 hours later, LIMA or its vehicle were intraarticularly administered (all procedures were performed under Isoflurane inhaled anesthesia). We evaluated knee perimeter, as a parameter of inflammation/swelling at time 0, 24 and 48h post MSU injection (REF). Blood samples were collected from the auricular artery 24 and 72 h after MSU administration. At sacrifice, knee synovial fluid and synovial membrane (SM) were collected. SM was cut into 2 equal pieces. Samples were stored at -80°C for molecular biology experiments or fixed in PFA 4% and embedded in paraffine for histopathological analysis.

Acute gout flare was induced after 2 weeks of adaptation to our facilities. Rabbits were housed in individual cages (0.50 m height, 0.6 m2 floor space) and exposed to a 12-hour light/dark cycle. To evaluate the possible undesirable effects of our treatments, we weighted animals daily. Rabbits were anesthetized with inhaled isoflurane and euthanized with an overdose of intracardiac pentobarbital (Ketamina+Xilacina). All the experiments were performed in accordance with the Animal Research Reporting of In vivo Experiments (ARRIVE) guidelines and with the National regulation and the Guidelines for the Care and Use of Laboratory Animals, drawn up by the National Institutes of Health (Bethesda, MS, USA). These procedures were approved by the Institutional Ethics and Animal Welfare Committee of IIS-FJD.

### Synovial fluid cell infiltration

We prepare a cell suspension of not more than 0.5×10⁶ cells/ml, and we loaded 150 µl of this suspension in each CytoSpin-cuvette. We spun at 800 rpm for 3 min. We stained the slides following the Panoptic kit instructions (254807, PanReac AppliChem) and we measured the number and type of cells from 3 aleatory fields.

### Evaluation of SM histological damage

Two 3 µm slides per sample were deparaffined and stained with hematoxylin and eosin for synovial histological damage scoring. A blinded evaluation of histopathology was done, and standardized Krenn score method was performed as previously described (Krenn V, Morawietz L, Burmester GR, Kinne RW, Mueller-Ladner U, Muller B, et al. Synovitis score: Discrimination between chronic low-grade and high-grade synovitis. Histopathology. 2006;49(4):358-64). Briefly, lining hyperplasia, tissue cell infiltration and stromal activation were independently evaluated using a subscale graded from 0 to 3 points. The total synovitis score was calculated summing partial grades, with a maximum of 9 points.

### RNA isolation from SM and RT-PCR

Total RNA isolation and semiquantitative RT-PCR were performed as described previously (Ref.). Gene expression was determined using the TaqMan probes listed below:
- IL-1β: On demand AI6RNOE, ThermoFisher
- IL-8: Oc03397858_m1, ThermoFisher
- ARG-1: Oc03397218_m1, ThermoFisher
- CD36: Oc03395926_m1, ThermoFisher
- GAPDH: Oc03823402_g1, ThermoFisher

### Determination of CRP in rabbit serum

Blood was recollected using vacutainer yellow tubes with separator gel and centrifuge for 20 min 2500 rpm at 4°C. C-reactive protein (CRP) levels were measured in the serum with a specific commercial enzyme-linked immunosorbent assay (ab157726, Abcam), following manufacturer's instructions.

### Statistical analysis

GraphPad Prism package (8.0 for Windows) was used for statistical analysis. To compare the evolution of joint perimeter between the different groups, two-way analysis of ANOVA variance was employed followed by Bonferroni's correction. For all other statistical evaluations, an overall one-way ANOVA test was undertaken and adopting a Bonferroni's correction. Quantitative data were expressed as mean ± SEM. P values less than 0.05 were considered significant.

### Example 1. LIMA treatment promotes macrophage phagocytic activity in vitro in higher extent.

We observed a significant decrease in THP-1 phagocytosis in the LXA4 and MAR1 group compared to vehicle at time 1 and 2 hours (Fig. 1A; Fig. 1B). These results are surprising given that the literature (see, e.g. Chiang et al. https://doi.org/10.1172/JCI129448 and Godson et al. https://doi.org/10.4049/jimmunol.164.4.1663) supports the proefferocytic effect of separate LXA4 and MAR1 on THP-1 macrophages. However, these studies usually involve undifferentiated (monocytic state) or PMA-differentiated THP-1 (M0), which generates a mild inflammatory phenotype, without the presence of M1 stimuli. In our case we decided to add IFN-y and LPS to favour a more inflammatory and aggressive phenotype that is very common in an acute-inflamed joint. In this way, we tested individual and combined SPMs treatment in a more similar scenario to that observed in pathology.

Remarkably, the combination of LXA4 and MAR1 in the LIMA group promoted a significant increase in phagocytic activity at all times compared to the rest of the groups (vehicle and separated LXA4 and MAR1) (Fig. 1A; Fig. 1B).These results evidence that LIMA treatment is the best approach to promote macrophage phagocytic activity *in vitro,* due to an unexpected synergistic effect derived from the specific combination of LXA4 with MAR1.

### Example 2. LIMA is the best treatment to mitigate neutrophil migration in vitro

To confirm the synergistic effect derived from the combination of LXA4 and MAR1 in resolving inflammation, we next evaluated neutrophil migration/recruitment 1- and 2-hours post treatment. At 1 hour, we did not observe any effect in the percentage of neutrophil migration in LXA4 and MAR1 groups vs vehicle (Fig. 2A). However, LIMA group elicit an early significative blockade of neutrophil migration at this time in comparison to the rest. At 2 hours, LIMA and LXA4 groups were the only ones able to inhibit the migration, while no change was observed in the MAR1 vs vehicle group.

These results demonstrate that, while LXA4 and MAR1 administered alone did not inhibit neutrophil migration at early times (1 h post treatment), the combination of both in the LIMA group caused a strong inhibition of neutrophilic chemotaxis at both early (1h) and late (2h) times, confirming the presence of an advantageous synergistic effect between LXA4 and MAR1, and establishing LIMA treatment as the best therapy to mitigate neutrophil migration *in vitro.* The effect of LIMA's treatment was quick, as it was observed in less than 20 hours after local administration. This rapid effect is especially desired in the treatment of diseases that cause acute inflammation outbreaks, such as acute joint pathologies, since the neutrophilic infiltrate starts in the first few minutes and grows exponentially for hours, which can lead to tissue damage and pain. The faster the compound of interest acts on this neutrophilic afference, the better the subsequent damage will be prevented.

Taken together, these *in vitro* results demonstrate that in two fundamental processes of acute inflammation resolution, phagocytosis (efferocytosis) and chemotaxis, LIMA is the most effective treatment. Therefore, the results of this combination were successfully validated *in vivo.*

### Example 3. LIMA reduces joint swelling and serum inflammation marker CRP.

It can be observed that 48 hours after the administration of MSU crystals, the untreated MSU group shows visible haemorrhagic leakage and swelling in comparison to Control group (Fig. 3A). MSU induced a marked increase in knee perimeter in a few hours, reaching its peak at 24 h after injections (Fig. 3B). Serum CRP concentration was also increased in MSU group at 24 h and continued elevated at 48 h vs Control group (Fig. 3C).

However LIMA injection evoked a visible reduction in haemorrhagic leakage (Fig. 3A), and a surprising and complete decrease in knee swelling intensity and duration at 24 hours, returning to baseline levels (Fig. 3B) and remaining so for up to 48 hours. Moreover, LIMA had an important positive effect on the systemic inflammation at 24 and 48 hours after MSU injection (Fig. 3C).

Our results highlight the *in vivo* ability of LIMA treatment to decrease knee swelling caused by MSU crystal injection and to reduce systemic inflammation.

### Example 4. LIMA treatment prevents histopathological lesions in the synovial membrane (SM).

In the MSU group, synovium histological alteration was observed 48 h after crystals injection (Fig. 4A), characterized by hyperplasia of lining layer, stromal activation with increased cellularity, irregular adipocytes and increased fibrotic component in the synovial stroma, as well as a boost of infiltrating cells, in comparison to Control group (Fig. 4B).

LIMA treatment induced a clear decrease in the histopathological lesions in the synovial membrane (Fig. 4A), with amelioration and return to Control levels of all parameters evaluated: lining hypertrophy, synovial stroma and accumulation of inflammatory cells (Fig. 4B)

Altogether, these results point out that LIMA administration can prevent synovial damage returning to baseline levels representing a significant improvement in the current management of the pathophysiology of acute arthritis.

### Example 5. LIMA inhibits neutrophilic recruitment in SM.

The injection of MSU crystals resulted in a marked significant increase in the number of PMNCs in the synovial fluid after 48 hours, which was accompanied by a decrease in the percentage of MNCs compared to the Control group (Fig. 5A; Fig. 5B).

In contrast, LIMA administration had a positive effect on the inhibition of neutrophilic chemotaxis, decreasing the number of PMNCs in the synovial fluid of the joint, and favoring the balance towards a higher percentage of MNCs. (Fig. 5A; Fig. 5B).

The inhibitory effect on neutrophil infiltrate exerted by LIMA is of utmost relevance since it can blunt the exacerbation of the acute response to an inflammatory insult.

### Example 6. LIMA reduces proinflammatory gene expression and stimulate pro-resolving pathway in the SM.

We observed a general increase in the expression of inflammatory genes associated with the gouty arthritis process in the MSU group at 48 hours, such as IL-1B or IL-8 (Fig. 6A). Treatment with LIMA restored the expression levels of these proinflammatory cytokines to control levels (Fig. 6A) in the SM.

In the case of Arg-1 and CD36, M2 polarization markers, injection of MSU crystals promotes a tendency to reduce their expression in comparison to Control (Fig. 6B), while LIMA administration appears to augment it.

Gene expression results support the anti-inflammatory and pro-resolving role of LIMA treatment, inhibiting the production of inflammatory cytokines and favoring the polarization towards a pro-efferocytic M2 phenotype.

### Example 7. We did not detect side effects under LIMA treatment.

We did not see any weight variation along the study in any of the groups. Furthermore, LIMA intra-articular administration was not associated with any macroscopic change in synovial structure. We can assume the lack of toxicity in rabbits in short-term treatment.

## Claims

1. A **composition** comprising a) Lipoxin A4, or a salt thereof, and b) Maresin-1, or a salt thereof.

2. The composition according to claim 1, wherein the composition is a pharmaceutical or a nutraceutical composition.

3. The composition according to any one of claims 1 or 2, **for use** in therapy.

4. The composition according to any one of claims 1 or 2, **for use** in the treatment, prevention, amelioration or reduction of inflammation or diseases that occur with inflammation.

5. The composition for use according to claim 4, wherein the disease is selected from the group consisting of gout, pseudogout, arthritis, arthritis associated with pyrophosphate deposits or other microcrystal arthropathies, traumatic arthritis, reactive arthritis, osteoarthritis, tendinitis, tenosynovitis, and rheumatoid arthritis.

6. The composition for use according to any one of claims 4 or 5, wherein the disease is caused by accumulation of microcrystals in one or more joints.

7. The composition for use according to claim 6, wherein the disease is gout, pseudogout, and/or chondrocalcinosis.

8. The composition for use according to any one of claims 4 to 7, wherein the disease is arthritis, preferably crystalline arthritis, most preferably gout, and wherein the composition is administered intraarticularly.

9. The composition for use according to any one of claims 4 to 8, wherein the Lipoxin A4, or a salt thereof, and the Maresin-1, or a salt thereof, are administered simultaneously or sequentially, in any order.

10. The composition for use according to any one of claims 4 to 9, wherein the Lipoxin A4, or a salt thereof, and the Maresin-1, or a salt thereof, are administered in a sole composition or in separate compositions.

11. The composition for use according to any one of claims 4 to 10, wherein the Lipoxin A4, or a salt thereof, and the Maresin-1, or a salt thereof, are administered several times.

12. The composition for use according to any one of claims 4 to 11, wherein the Lipoxin A4, or a salt thereof, and the Maresin-1, or a salt thereof, are administered in a dosage capable of providing a pro-resolution of inflammation effect in an inflamed area.

13. The composition for use according to claim 12, wherein the pro-resolution of inflammation effect comprises one or more of: reducing or stopping neutrophil infiltration, reducing the concentration of pro-inflammatory cytokines (such as IL-10, TGFβ, etc), promoting the synthesis of anti-inflammatory cytokines and the release of growth factors that allow tissue reconstruction inducing the healing process and the recovery of the inflamed tissue, stimulating non-phlogistic recruitment of monocytes, stimulating macrophage polarization to an anti-inflammatory phenotype (M2 phenotype), enhancing macrophage phagocytosis of apoptotic neutrophils, increasing the exit of phagocytes from the inflammatory site to the lymphatics; and/or stimulating mucosal defence and the removal of cell debris in an inflamed area.

14. A **kit of parts** comprising a) Lipoxin A4, or a salt thereof, and b) Maresin-1, or a salt thereof, in the form of one or more recipients.
